# EUROPÄISCHE PATENTSCHRIFT

(11) **EP 1 838 293 B1**
(45) Veröffentlichungstag und Bekanntmachung des Hinweises auf die Patenterteilung: **15.10.2008**
(21) Anmeldenummer: 06700744.3
(22) Anmeldetag: 06.01.2006
(51) Int. Cl.: A61K 9/48

(54) **ZWEITEILIGE KAPSEL MIT VORVERSCHLUSS ZUR AUFNAHME VON PHARMAZEUTISCHEN ZUBEREITUNGEN F]R PULVERINHALATOREN**
TWO-PIECE CAPSULE COMPRISING A PRELIMINARY CLOSURE FOR ACCOMMODATING PHARMACEUTICAL PREPARATIONS FOR POWDER INHALERS
CAPSULE EN DEUX PARTIES A FERMETURE PREALABLE DESTINEE A RECEVOIR DES PREPARATIONS PHARMACEUTIQUES POUR DES INHALATEURS A POUDRE

(30) Priorität: 11.01.2005 DE 102005001332
(43) Veröffentlichungstag der Anmeldung: 03.10.2007
(73) Patentinhaber: Boehringer Ingelheim Pharma GmbH & Co.KG, 55216 Ingelheim am Rhein (DE)
(72) Erfinder: KUEHN, Torsten, 55437 Appenheim (DE); KUHN, Rolf, 55218 Ingelheim Am Rhein (DE); METZGER, Burkhard, 55218 Ingelheim Am Rhein (DE); HOELZ, Hubert, 55413 Oberheimbach (DE); LUSTENBERGER, Stefan, 55457 Gensingen (DE); WACHTEL, Herbert, 55218 Ingelheim Am Rhein (DE)
(74) Vertreter: Hammann, Heinz
(86) Internationale Anmeldenummer: PCT/EP2006/050064
(87) Internationale Veröffentlichungsnummer: WO 2006/074982

(56) Entgegenhaltungen:
- EP-A- 0 143 524
- WO-A-00/07572
- WO-A-20/04062716

## Beschreibung

Die Erfindung betrifft neue zweiteilige Kapseln zur Aufnahme von pharmazeutischen Zubereitungen zur Verwendung in Pulverinhalatoren, die einen Hauptverschluss und einen Vorverschluss aufweisen.

### Stand der Technik

Kapseln mit pharmazeutischen Zubereitungen werden vielfältig in der Therapie und Diagnose von Krankheiten eingesetzt. Die Kapseln können oral verabreicht werden oder kommen in bestimmten medizinischen Vorrichtungen wie Pulverinhalatoren zum Einsatz. In der Regel bestehen die Kapseln aus zwei Teilen, einem Kapselkörper (Körper) und einer Kapselkappe (Kappe), die teleskopartig ineinander geschoben werden. Aber auch mehrteilige Kapseln sind bekannt. Die Kapseln bestehen meistens aus Gelatine, insbesondere Hartgelatine. Für einige spezielle Anwendungen bestehen die Kapseln zuweilen auch aus für den Menschen gut verdaulichen, wasserlöslichen Kunststoffen, um z.B. bei oraler Verabreichung den Wirkstoff in bestimmten Kompartimenten des Magen-Darm-Trakts freizusetzen.

Die EP 1100474 offenbart Kunststoffkapseln, die aus einem Kapselkörper und einer Kapselkappe bestehen, die beide aus dem gleichen nicht-wasserlöslichen, hydrophoben Kunststoff bestehen und die so miteinander verbunden werden können, daß ein stabiler, abgeschlossener Hohlraum von definiertem Volumen gebildet wird. Insbesondere handelt es sich bei dem Kunststoff um Polyethylen. Die Kapsel kann Rastelemente aufweisen, die die Kapselkappe fest mit dem Kapselkörper verbinden. Die Kapseln sind zur Verwendung in Pulverinhalatoren bestimmt. Die vorliegende Erfindung betrifft eine Weiterentwicklung dieser Kapseln. Daher wird hiermit explizit auf den Inhalt der EP 1100474 in vollem Umfang Bezug genommen.

Dementsprechend sind auch die erfindungsgemäßen Kapseln zur Verwendung für jede Art von Pulverinhalator, die mit Kapseln als Wirkstoffreservoir arbeiten vorgesehen und geeignet. Als Beispiel für derartige Pulverinhalatoren seien genannt: Inhalatoren wie sie unter den Markennamen Spinhaler^{®}, Rotahaler^{®}, Aerolizer^{®}, Flowcaps^{®}, Turbospin^{®}, AIR DPI^{®}, Orbital^{®}, Directhaler^{®} bekannt sind und/oder in DE 33 45 722, EP 0 591 136, DE 43 18 455, WO 91/02558, FR-A-2 146 202, US-A-4 069 819, EP 666085, EP 869079, US3991761, W099/45987 beschrieben werden. Besonders jedoch sind die Kapseln vorgesehen zur Verwendung in einem Inhalator der Marke HandiHaler^{®}, wie er z.B. in der EP 1342483 offenbart wird.

Bei dem Gerät der Marke HandiHaler^{®} handelt es sich um einen Inhalator, in dem das Bernoulli-Prinzip Anwendung findet. Den Bernoulli-Inhalatoren ist gemeinsam, dass der auszubringende Wirkstoff in einer zylinderartigen Kapsel gelagert wird und diese Kapsel in eine Kapselkammer des Inhalators eingesetzt wird. Die Kapselkammer ist dabei meist ebenfalls zylindrisch ausgebildet, wobei sie etwas länger und etwas größer im Durchmesser als die Kapsel ist, so dass die Kapsel in ihr zwar sowohl vertikal (= axial) als auch horizontal (= radial) vibrieren kann, dabei aber im wesentlichen parallel zur Kammerachse ausgerichtet bleibt. Die Kapselkammer weist im Bereich eines der beiden Enden einen Lufteinlass und im Bereich des anderen Endes eine Luftauslassöffnung auf. Der Luftauslass (Luftkanal) führt zu einem Mundstück. Im Rahmen der vorliegenden Erfindungsbeschreibung definiert die so von der Kapselkammer über den Luftkanal zum Mundstück aufgespannte Richtung die Längsachse und damit die axiale Richtung. Die dazu senkrechte Richtung definiert die vertikale oder radiale Richtung.

Zum Ausbringen des aktiven Kapselinhalts wird zunächst die Kapsel an üblicherweise zwei Stellen am längsseitigen Mantel geöffnet. In der Regel finden sich die Öffnungen in der Nähe der beiden längsseitigen Enden der Kapsel. Wird nun in der Kapselkammer ein Luftstrom vom Lufteinlass zum Luftauslass erzeugt, führt dieser entlang der Längsachse der Kapsel und bewirkt dabei zweierlei: Zum einen wird die Kapsel durch den Luftstrom hauptsächlich entlang ihrer Längsachse bewegt. Dabei kann sie auch in einem geringen Bereich vibrieren. Zum anderen erzeugt die an den beiden Kapselöffnungen entlang strömende Luft vor den Kapselöffnungen gegenüber dem Kapselinneren einen Unterdruck, so dass das in der Kapsel befindliche Pulver vom Luftstrom mitgerissen und dabei zerstäubt wird.

Die für solche Inhalatoren üblicherweise verwendeten Kapseln bestehen aus zwei becherartigen Teilen, die teleskopartig ineinander einsteckbar sind. Die äußere Form einer solchen zusammengesteckten Kapsel ist die eines geschlossenen Zylinders mit halbkugelförmigen Enden. Der Zylinder weist eine Längsachse und eine Querachse auf. Die Längsachse ist dabei diejenige Achse, die parallel zu den Erzeugenden des Zylindermantels liegt. Die Längsachse ist länger als die Querachse, so dass der Längsschnitt der Kapsel eine ovale, der Querschnitt eine kreisförmige Geometrie aufweist.

Der bereits erwähnte und im Rahmen der vorliegenden Erfindung bevorzugte HandiHaler^{®} besteht aus a) einem nach oben hin offenen, becherförmigen Unterteil, b) einer Platte, welche die Öffnung des Unterteils bedeckt und senkrecht zu der eine Kapselkammer der oben beschriebenen Art ausgebildet ist, wobei an der Kapselkammer ein gegen eine Feder beweglicher Knopf vorgesehen ist, der zwei geschliffene Nadeln zum Öffnen der Kapsel aufweist, c) einem Oberteil mit einem Mundrohr, das - ein Pulveraerosol leiten könnend - mit der Kapselkammer verbunden ist und d) einem Deckel. Dabei sind die Elemente a), b) c) und d) durch ein gemeinsames Scharnierelement miteinander verbunden, so dass sie gegeneinander klappbar bewegt werden können.
Daneben beschreibt diese Patentanmeldung einen Kapselhalter, wobei der Kapselhalter als Loch in der Platte b) ausgebildet sein kann und am Rand Rippen aufweist. Die Kapsel wird in diesen Kapselhalter zum Zweck der Bevorratung eingeklemmt.

Weitere Bernoulli-Inhalatoren werden in der DE 3345722 und der WO 91/02558 offenbart.

Beim Herstellen und anschließenden Befüllen von Kapseln gemäß der EP 1100474 werden zunächst die Kapseln maschinell hergestellt, provisorisch verschlossen, anschließend zu den Befüllungsanlagen transportiert, dort wieder geöffnet, gefüllt und schließlich endgültig verschlossen. Für den provisorischen Verschluss der Kapsel wird in der Regel die Kapselkappe lose auf den Kapselkörper aufgesteckt. Während des weiteren Prozedere kann es jedoch passieren, dass die Kapselkappe von dem Kapselkörper abfällt oder sich so öffnet, dass eine Verschmutzung des Kapselinneren möglich wird. Solche geöffneten oder verschmutzten Kapseln müssen dann vor dem Befüllungsvorgang mit der pharmazeutischen Formulierung ausgemustert werden.

### Beschreibung der Erfindung

Die vorliegende Erfindung löst dieses Problem, indem eine Kapsel der vorbeschriebenen Art geschaffen wird, die neben einem endgültigen Verschlussmittel, dem Hauptverschluss auch noch ein provisorisches Verschlussmittel aufweist, den Vorverschluss.

Daher ist es eine Aufgabe der vorliegenden Erfindung Kapseln zu schaffen, die sich einfach herstellen lassen, in einfacher Weise provisorisch verschließbar sind, jedoch leicht wieder geöffnet werden können, und die schließlich auch endgültig so verschlossen werden können, dass sie bevorzugt nur unter Beschädigung der Kapsel wieder geöffnet werden können.

Eine weitere Aufgabe der Erfindung besteht darin, derartige Kapseln zu schaffen, bei der für den provisorischen Verschluss und den endgültige Verschluss möglichst wenig Strukturelemente an der Kapselkappe oder dem Kapselkörper ausgebildet werden müssen.

Wieder eine andere Aufgabe besteht darin, eine derartige Kapsel zu schaffen, bei der für den provisorischen und/oder endgültigen Verschluss der Kapsel keine Vorzugsorientierung zwischen Kapselkörper und Kapselkappe notwendig sind, die gegenüber einem einfachen Zusammenstecken der beiden Teilelemente über den jeweils offenen Bereich derselben hinausgehen.

### Detaillierte Beschreibung der Erfindung

Die erfindungsgemäße Kapsel besteht aus zwei Teilen, einem Kapselkörper (Körper) und einer Kapselkappe (Kappe), die so miteinander verbunden werden können, dass ein stabiler, abgeschlossener Hohlraum von definiertem Volumen gebildet wird, der die pharmazeutische Formulierung beinhaltet. Die Dimension der Kapsel ist derart, dass sie in gängigen mit Kapseln bestückten Pulverinhalatoren eingesetzt werden können, wie sie z.B. in den Patentschriften DE 33 45 722 (Inhalator Ingelheim M), EP 0 591 136 (Inhalator Ingelheim) oder in der EP 1342483 (HandiHaler®) beschrieben sind.

Die Mäntel der Kappe und des Körpers beschreiben einen hohlen Zylinders mit rundem Querschnitt, wobei die jeweilige Oberseite offen und die Unterseite geschlossen ist. Die geschlossenen Kapsel ist damit außen konisch und innen zylindrisch. Die geschlossene Unterseite kann flach oder konvex sein oder eine andere werkzeugbedingte Form haben. Die Elongation der geschlossenen Kapsel (Abstand vom geschlossenen Ende des Körpers zum geschlossenen Ende der Kappe in Relation zum größten Durchmesser der geschlossener Kapsel) ist größer 1.

Als Kunststoffmaterial für die Kapsel kommen alle pharmazeutisch akzeptablen Kunststoffe in Frage, die spritz- oder blasformtechnisch sowie tiefziehtechnisch durch Thermoformen verarbeitet werden können und/oder Kunststoffe für deren Verarbeitung zur Kapselkappe oder zum Kapselkörper kein Formtrennmittel notwendig ist, das ein Anhaften der Inhaltsstoffe an die Kapselwand bewirken kann. Der Kunststoff sollte auch keine ausgeprägte Adhäsion für pharmazeutisch-chemische Stoffe, insbesondere für Partikel mit lungengängiger Größe aufweisen. Die bevorzugte Shorehärte D der Materialien liegt zwischen 10 und 85, bevorzugt zwischen 55 und 75, besonders bevorzugt 60 bis 70. Das Material sollte auch so sein, dass eine Kunststoffkapsel entlang der Längsache einer Kraft bis zu 20 N standhält. Außerdem sollte die Wand der Kapsel eine Durchlässigkeit für Wasserdampf von weniger als 1,3 x 10⁻¹⁴ kg/(m² s Pa), bevorzugt von 1,5 x 10⁻¹⁶ bis 2 x 10⁻¹⁶ kg/(m² s Pa) aufweisen. Die Schmelzeviskosität MFR (melt flow rate) liegt bevorzugt zwischen 40 und 65 g/10 Minuten, bevorzugt bei 45 - 59 g/10 Minuten und besonders bevorzugt bei 52 g/10 Minuten.

In bevorzugten Ausführungen ist der Kunststoff Polyethylen, insbesondere Polyethylen mit einer Dichte zwischen 900 und 1000 kg/m³, bevorzugt zwischen 960 und 970 kg/m³ (highdensity Polyethylen). Daneben sind auch Polycarbonat, Polyester, Polypropylen, Polyethylenterephthalat, Polyutethan geeignet. Bevorzugt ist Poylethylen. Außerdem können geschäumte Kunststoffe verwendet werden, beispielsweise solche, wie sie z.B. unter dem Markennamen Hydrocerol^{®} bekannt sind. Bei diesen Materialien handelt es sich um Kunststoffe, die während des Verarbeitungsvorgangs (Spritzguss) durch zugesetzte oder freigesetzte Schäumungsmittel aufgeschäumt werden. Als Schäumungsmittel können beispielsweise verwendet werden Azadicarbonamid (ADC), 4,4'-Oxybis(benzolsulfonylhydrazid) (BSH), 5-Phenyltetrazol (5-PT), p-Tuluylsulfonylsemicarbazid (TSS), p-Tuluylsulfonylhydrazid (TSH), verschiedene Citrate, Zitronensäure, Carbonate, wie Natriumdicarbonat und andere aus dem Stand der Technik bekannte Schäumungsmittel.

In einer bevorzugten Ausführungsform haben die Kappe und der Körper die Form eines auf der einen Seite offenen Zylinders mit rundem Querschnitt und konvexer, nahezu halbkugelförmiger geschlossener anderer Seite (Unterseite) und bestehen beide aus highdensity Polyethylen mit einer Dichte zwischen 950 und 1000 kg/m³.

Die Kapselgrößen sind an die jeweiligen Inhalatoren angepasst. Beispiele für Kapseldimensionen sind:
- Länge der Kapselkörper: von ca. 22 bis ca. 9 mm, bevorzugt: 22,2 ±0,46 mm; 20,22 ±0,46 mm; 20,98 ±0,46 mm; 18,4 ±0,46 mm; 16,61 +0,46 mm; 15,27 ±0,46 mm; 13,59 ±0,46 mm; 13,1 ± 0,1 mm;_12,19 +0,46 mm; 9,3 ±0,46 mm.
- Länge der Kapselkappe: von ca. 13 bis 6 mm, ca. bevorzugt: 12,95 ±0,46 mm; 11,74 ±0,46 mm; 11,99 ±0,46 mm; 10,72 ±0,46 mm; 9,78 ±0,46 mm; 8,94 ± 0,46 mm; 8,54 ± 0,1 mm;_8,08 ±0,46 mm; 7,21 ±0,46 mm; 6,2 ±0,46 mm.
- Äußerer Durchmesser der Kapselkörper: von ca. 10 bis ca. 4 mm, bevorzugt: 9,55 mm; 8,18 mm; 7,36 mm; 7,34 mm; 6,63 mm; 6,07 mm; 5,57 ± 0,06 mm; 5,05 mm; 4,68 mm.
- Äußerer Durchmesser der Kapselkappen: von ca. 10 bis ca. 4 mm, bevorzugt: 9,91 mm; 8,53 mm; 7,66 mm; 7,64 mm; 6,91 mm; 6,35 mm; 5,83 ± 0,06 mm; 5,32 mm; 4,91 mm.
- Gesamtlänge der geschlossenen Kapsel: von ca. 27 bis ca. 11 mm, bevorzugt: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3 ±0,3 mm; 11,1 ±0,3 mm.
- Kapselvolumina: von ca. 1, 4 bis ca. 0,1 ml, bevorzugt: 1,37 ml; 0,95 ml; 0,78 ml; 0,50 ml; 0,37 ml; 0,30 ml; 0,24 ml; 0,21 ml; 0,13 ml.
- Gewicht der Kapseln: von ca. 170 mg bis ca. 20 mg, bevorzugt zwischen 80 und 125 mg, bevorzugte Einzelwerte: 163 mg; 118 mg; 110 mg; 105 mg, 100 mg, 96 mg; 76 mg; 61 mg; 48 mg; 38 mg; 28 mg.

Erfindungsgemäß sind Kappe und Körper, die wie zwei komplementäre Bauteile über ihre offenen Seiten ineinander gesteckt werden können, mit einem Vor- und einem Hauptverschluss versehen.

Dabei besteht der Vorverschluss aus einer ersten, im Bereich der Öffnung peripher, den Außenmantel des Kapselkörpers als Ring umlaufenden Vertiefung und einer ersten peripher, den Innenmantel der Kapselkappe ebenfalls im Bereich deren Öffnung als Ring umlaufenden Erhebung, die ggf. segmentiert ist. Die Vertiefung und die Erhebung passen dabei wie Feder und Nut ineinander.

Die folgenden Angaben zu Größen beziehen sich bevorzugt auf eine Kapsel der Größe 3. Diese ist gekennzeichnet durch: Länge des Kapselkörpers: 13,1 ± 0,1 mm, Länge der Kapselkappe: 8,54 ± 0,1 mm, Gesamtlänge der Kapsel: 15,9 ± 0,3 mm, Außendurchmesser des Körpers: 5,57 ± 0,06 mm, Außendurchmesser der Kappe: 5,83 ± 0,06 mm.

Die Vertiefung hat bevorzugt eine Tiefe von 0,03 bis 0,1 mm, besonders bevorzugt 0,05 bis 0,08 mm, stärker bevorzugt 0,065mm. Ihre Breite beträgt bevorzugt 0,1 bis 0,25 mm, bevorzugt 0,15 bis 0,2 mm, besonders 0,18 mm.

Die Erhebung hat eine Höhe von 0,04 bis 0,08 mm, bevorzugt 0,06 mm. Dabei ist es für den Vorverschluss nicht notwendig, dass der Federteil den Nutteil vollständig ausfüllt. Die Erhebung kann dabei segmentiert sein. Die Breite der Erhebung beträgt 0,2 bis 0,8 mm, bevorzugt 0,3 bis 0,6 mm.

Die Segmentierung kann alternativ nur an dem erhabenen Ring ausgebildet werden und dabei z.B. als Kette von punktförmigen Erhebungen ausgebildet sein, bis hin zu einer einzigen, punktförmigen Erhebung. Bevorzugt kann eine Teilsegmentierung in drei Segmente sein, die durch nicht erhabene Teilbereiche unterbrochen wird. Die Segmente haben bevorzugt eine Länge von 4 mm. Die Länge der nicht erhabenen Teilbereiche beträgt bevorzugt 1.3 mm. Die Länge der Segmente kann aber naturgemäß frei gewählt werden, wodurch die Kraft, mit der die Segmente in den komplementären Vertiefungen sitzen gesteuert werden kann. Länge bedeutet hierbei die Ausdehnung des Segments senkrecht zur Breite. Die Breite ist die Ausdehnung parallel zur Mittelachse, die von der Öffnung des Kapselelements (Kappe oder Körper) zum geschlossenen Ende weist. Alternative Ausführungsformen mit wenigstens einer oder zwei punktförmigen Segmenten kürzerer Länge werden in den Figuren angedeutet.

Der endgültige Verschluss (Hauptverschluss) besteht wiederum aus der peripher, den Außenmantel des Kapselkörpers als Ring umlaufenden ersten Vertiefung, wie er bereits in Zusammenhang mit dem Vorverschluss beschreiben worden ist und einer von der Öffnung aus gesehen hinter der ersten Erhebung liegenden zweiten peripher, den Innenmantel der Kapselkappe als Ring umlaufenden Erhebung, die ebenfalls ggf. segmentiert sein kann. Die Vertiefung und die Erhebung passen dabei ebenfalls wie Feder und Nut ineinander. Auch hier ist es nicht notwendig, dass der Federteil den Nutteil vollständig ausfüllt.

Die zweite Erhebung hat eine Höhe von 0,08 bis 0,13 mm, bevorzugt 0,10 -0,11 mm. Die Breite der Erhebung beträgt 0,2 bis 0,8 mm, bevorzugt 0,3 bis 0,6 mm.

Wichtig ist, dass diese zweite Erhebung so ausgebildet ist, dass sie die Vertiefung vollständiger ausfüllt, also mehr Reibungsfläche bietet als die erste Erhebung. Dafür kann die zweite Erhebung weniger segmentiert sein, bei gleicher Höhe und Breite, sie kann höher oder breite sein als die erste oder es handelt sich um Kombinationen aus diesen Varianten. Wichtig ist jedoch, dass auch die zweite Erhebung vollständig von der komplementären Vertiefung an dem anderen Teilelement der Kapsel aufgenommen werden kann. Bevorzugt ist die zweite Erhebung höher, als die erste Erhebung. Der Vorverschluss ist bevorzugt so gestaltet, dass die Kraft zum Öffnen durch Auseinaderzeihen der beiden Kapselelemente (Kappe und Körper) zwischen 0,1 und 3 N liegt, bevorzugt bis zu 2,5 N. Der Hauptverschluss kann bevorzugt durch Auseinanderziehen nicht zerstörungsfrei geöffnet werden, zumindest ist der zum Öffnen notwendige Kraftaufwand höher als die bis zu 3 N zum Öffnen des Vorverschlusses.

Damit die erste Erhebung nicht auf dem Mantel der Kapselkappe aufsteht, wenn die zweite Erhebung in die erste Vertiefung einrastet, ist hinter der ersten Vertiefung (gesehen aus Richtung der Öffnung) eine zweite ringförmige Vertiefung ausgebildet, deren Dimensionen denen der ersten Vertiefung entsprechen kann. Die bevorzugte Tiefe dieser zweiten Vertiefung beträgt jedoch 0,02 - 0,09 mm, besonders bevorzugt 0,05 - 0,06 mm. Ihre Breite beträgt bevorzugt 0,1 bis 0,25 mm, bevorzugt 0,1 bis 0,15 mm, besonders 0,13 mm.

Die Erhebungen können auch auf dem Außenmantel des Kapselkörpers und die Vertiefungen auf dem Innenmantel des Kapselkappe ausgebildet sein.

In einer weiteren Ausführungsform ist auf der Außenseite des Körpers zusätzlich ein Wulst ausgebildet, der senkrecht zu der Verbindungsachse zwischen Kappe und Körper ringsum den Körper läuft. Der Wulst dient als Stopper für die Kapsel, wenn diese über den Körper gesteckt wird, um ein Durchstoßen der Kappe mit dem Körper zu verhindern. Der Bereich zwischen offenem Ende des Körpers und dem Wulst entspricht dem Bereich des Körpers über den die Kappe geschoben werden kann. Der Wulst ist so auf dem Körper lokalisiert, dass die Kappe weit genug über den Körper geschoben werden kann, damit die Verschlussmittel wie beschrieben einrasten können. Demgemäß befindet sich der Wulst hinter der zweiten ringförmigen Vertiefung bzw. Erhebung. Die Seite des Wulstes, die zum offenen Ende des Körpers zeigt, steht als senkrechte Kante so auf der Außenwand des Körpers, dass die Kappe beim Verschließen nicht über den Wulst hinweg geschoben werden kann. Die Seite des Wulstes, die zum geschlossenen Ende des Körpers weist, kann in Form einer nahezu rechtwinkeligen Kante ausgebildet sein oder sich zum geschlossenen Ende des Körpers hin verflachen. Die Ausbildung einer nahezu rechtwinkeligen Kante kann bei einer losen Einpassung der Kapsel in einen Kapselhalter von Vorteil sein, die Variante mit sich verflachendem Wulst bei einer festen Einpassung. Der Wulst kann durchgängig oder unterbrochen sein.

In einer bevorzugten Ausführungsform verflacht sich der Wulst kontinuierlich zum geschlossenen Ende des Körpers und steht mit seiner zum offenen Ende des Körpers orientierten Seite senkrecht auf dem Kapselkörper auf. Dabei ist die Höhe der so gebildeten Kante derart, dass die Kante im geschlossenen Zustand der Kapsel nicht über die Kapselkappe hinaus ragt. Der Übergang von Kapselkappe zu Kapselkörper kann plan sein, ist aber vorzugsweise abgesetzt, d.h. der Außendurchmesser der Kappe ist größer als der größte Außendurchmesser des Körpers, bzw. des Wulstes.

Die Stärke der Wände der Kappe und des Körpers können über den Gesamtbereich variieren. So ist die Wandstärke in der Regel in den abgerundeten Bereichen der Kappe oder des Körpers oder an der Stelle des Körpers, an der der Wulst ausgebildet ist größer als in den Bereichen, in denen die Wände geradlinig verlaufen. In einer Ausführungsform haben die Wände der Kappe und des Körpers eine Dicke von 0,1 mm bis 0,5 mm.

In einer möglichen Ausführungsform sind an der sichtbaren Außenseite der geschlossenen Kapsel Noppen ausgebildet, in einer anderen drei oder mehr Rippen, die parallel zur Längsachse der Kapsel verlaufen. Der Vorteil dieser Einrichtungen besteht darin, dass die Kapsel aus einer Kapselhalterung, wie sie z.B. in den oben genannten Pulverinhalatoren verwendet werden können, so herausgenommen werden kann, dass sie nicht beschädigt wird oder aufgeht. Ggf. können die Rippen auch als umlaufende, spiralförmige Erhebungen ausgebildet sein.

Weitere äußere Gestaltungsmöglichkeiten für die Kapsel können dem Stand der Technik entnommen werden.

Um im geschlossenen Zustand der gefüllten Kapsel eine bessere Abdichtung zwischen Kappe und Körper zu erreichen, kann die Nahtstelle zwischen Kappe und Körper zum Verschließen verschweißt, verklebt oder banderoliert werden, wodurch die Wasserdampfpermeabilität auf bis zu einem Zehntel abnimmt. Alternativ kann die gesamte Kappe mit einem durchgehenden Schutzfilm überzogen werden. Methoden zum Verschweißen der Kunststoffkapsel sind in der EP 1414639 offenbart. Derartige Verschweißmethoden umfassen das Verschweißen mittels Laser, Heißluft, Lötkolben etc.

In einer weiteren bevorzugten Ausführungsform kann der Spalt mit einem Füllstoff verschlossen werden. Als Füllstoff für ein solches Verfüllen des Spalts eigenen sich pharmazeutisch zulässige Füllmaterialien.

Gegebenenfalls kann es erwünscht sein, den Außenmantel der Kapsel zu beschriften. Dies kann durch herkömmliche Beschriftung mittels Tintenstrahldruck u.ä. vorgenommen werden. Alternativ dazu kann die Beschriftung nachträglich auch als Gravur aufgebracht werden oder die Beschriftung wird als Gravur oder Relief in die Spritzgießform eingearbeitet. Bei letzterem kann die dafür vorgesehene Schriftfläche abgeflacht oder anderweitig hervorgehoben ausgebildet sein. Die eingravierten oder reliefartigen Schriftzeichen können ihrerseits als Vertiefung oder Erhebung von z.B. 20 Mikrometern auf der Oberfläche ausgebildet sein. Die Beschriftung kann wahlweise auf dem Oberteil oder auf dem Unterteil oder beiden Elementen ausgebildet sein, bevorzugt ist die Beschriftung nur auf dem Unterteil ausgebildet. Durch eine unterschiedliche Strukturierung der Buchstaben, Ziffern oder Symbole kann die Kapsel mit Informationen zum Inhalt oder der Herkunft versehen werden.

Aus der Beschreibung wird ersichtlich, dass die erfindungsgemäße Kapsel geeignet ist, pulverförmige und zur Inhalation geeignete pharmazeutische Formulierung aufzunehmen. In einer besonderen Verwendungsform enthält die Kapsel zumindest einen der nachfolgend aufgeführten Wirkstoffe oder ggf. Kombinationen daraus:
Analgetika, Antiallergika, Anticholinergika, (Antimuskarinika), Antihistaminika, Antiifektiva, Antitussiva, Betamimetika, Bronchodilatoren, EGFR-Hemmer, LTD4-Antagonisten, PDE-IV-Inhibitoren, Steroide usw.

### Beispiele für Analgetika sind:

Codein, Dihydromorphin, Ergotamin, Fentanyl. Morphin; Diltiazem.
Beispiele für Antiallergika/Antihistaminka sind:
Cromoglicat, Dinatriumeromoglicat, Epinastin, Ketotifen, Nedocromil, Methapyrilen.

### Beispiele für Anticholinergika sind:

Tiotropiumsalzen, Oxitropiumsalzen, Flutropiumsalzen, Ipratropiumsalzen, Glycopyrroniumsalzen, Trospiumsalzen, wobei als Gegenion (Anion) jeweils bevorzugt Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten ist. Ipratropiumbromid, Oxitropiumbromid, Tiotropiumbromid, Tiotropiumbromid-Monohydrat, Trospiumchlorid sind jeweils bevorzugt. Die Verbidnungen können ggf. auch als Solvate, z.B. Hydrate verwendet werden.

### Beispiele für Antiinfektva sind:

Cephalosporin, Penicilline, Streptomycin, Sulphonamide, Tetracycline, Ppentamidin. Beispiele für Antitussiva sind Noscapine; Ambroxol.
Beispiele für Betamimetika sind:
Bambuterol, Bitolterol, Carbuterol, Clenbuterol, Fenoterol, Formoterol, Hexoprenalin, Ibuterol, Isoprenalin, Metaproterenol, Phenylephrin, Pirbuterol, Procaterol, Reproterol, Salbutamol, (als freie Base oder Sulfat), Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol , 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol oder 1-(4-Ethoxycarbonylamino-3-cyano-5-fluorophenyl)-2-(tert.-butylamino)ethanol.

### Beispiele für EGFR-Hemmer sind:

4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin, 4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-l-yl]amino}-7-cyclopentyloxy-chinazolin, 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(*R*)-(1-Phenylethyl)amino]-6-({4-[N-(2-methoxy-ethyl)-N-ethyl-amino]-1-oxo-2-buten-l-yl}amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluorphenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methylpiperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethylamino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

### Beispiele für LTD4-Antagonist sind:

Montelukast, 1-(((R)-(3-(2-(6,7-Difluoro-2-quinolinyl)ethenyl)phenyl)-3-(2-(2- hydroxy-2-propyl)phenyl)thio)methylcyclopropan-essigsäure, 1-(((1(R)-3(3-(2-(2,3-Dichlorothieno[3,2-b]pyridin-5-yI)-(E)-ethenyl)phenyl)-3-(2-(1-hydroxy-1-methylethyl)phenyl)propyl)thio)-methyl)-cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

### Beispiele für PDE-IV-Inhibitoren sind:

aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(*tert-*butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

### Beispiele für Steroide sind:

Prednisolon, Prednison, Butixocortpropionat, RPR-106541, Flunisolid, Beclomethason, Beclometason-17, 21-Dipropionat, Betamethasonvalerat, Betamethasonadamantoat, Triamcinolon, Budesonid, Flunisolid, Fluticason, Fluticason-propionat, Mometason, Mometason furoat, Ciclometason, Ciclesonid, Deflazacort, Rofleponid, ST-126, Dexamethason, Dexamethason-21-isonicotinat, Dexa- methasonisonicotinat, 6alpha,9alpha-Difluoro-17alpha-[(2-furanylcarbonyl)oxy]-11beta-hydroxy-16alpha-methyl-3-oxo-androsta-1,4-dien-17beta-carbothionsäure (S)-fluoromethylester und 6alpha,9alpha-Difluoro-11beta-hydroxy-16alpha-methyl-3-oxo-17alpha-propionyloxy-androsta-1,4-dien-17beta-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt sind Anticholinergika, insbesondere Tiotropium. Letzteres liegt ganz besonders bevorzugt als Tiotropiumbromid-Monohydrat vor. Daher betrifft ein Aspekt der Erfindung den erfindungsgemäßen Blister enthaltend Tiotropiumbromid-Monohydrat.

Die genannten Wirkstoffe können auch in Form von anderen pharmakologisch verträglichen Salzen, Additionsprodukten, Solvaten etc. eingesetzt werden.

Die Erfindung wird bevorzugt für Behälter mit den genannten Wirkstoffen oder Kombinationen verwendet, ist jedoch nicht auf die genannten Wirkstoffe beschränkt. Die mit den Arzneitstoffformulierungen befüllten Kapseln können zum weiteren Schutz vor Feuchtigkeit u.ä. zur Lagerung in Blister, Taschen o.ä. eingeschweißt oder in Flaschen und anderen Behältnissen gelagert werden. Besonders geeignet sind Blister und Taschen aus Aluminiumfolien oder Laminatfolien aus die bevorzugt auch eine Aluminiumschicht aufweisen.

### Beschreibung der Abbildungen

Die Abbildungen zeigen bevorzugte Ausführungsformen der erfindungsgemäßen Kapsel, dienen jedoch nur zur Illustration ohne den Umfang der Erfindung einzuschränken.
Fig. 1 zeigt eine bevorzugte Ausführungsform des Kapselkörpers im lateralen Querschnitt.
Fig. 2 zeigt eine bevorzugte Ausführungsform der Kapselkappe mit segmentiertem Vorverschluss im lateralen Querschnitt.
Fig. 3 zeigt eine bevorzugte Ausführungsform der Kapselkappe mit segmentiertem Vorverschluss im lateralen Querschnitt.
Fig. 4 zeigt eine bevorzugte Ausführungsform der Kapselkappe mit nicht-segmentiertem Vorverschluss im lateralen Querschnitt.
Fig. 5 zeigt den HandiHaler^{®}.

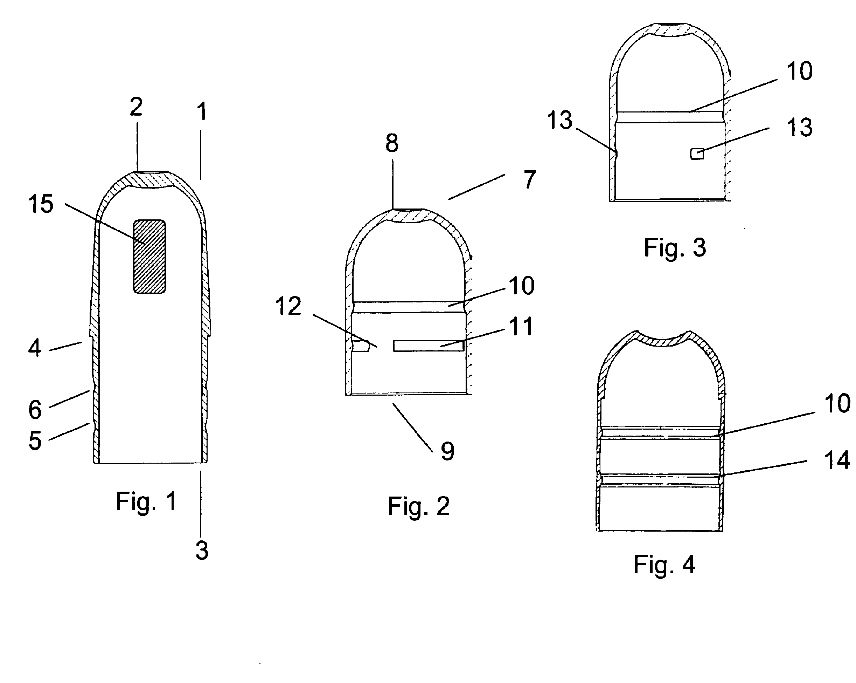

In Figur 1 ist eine Ausführungsform des erfindungsgemäßen Kapselkörpers **1** im Querschnitt gezeigt. Der Kapselkörper **1** weist eine konvexe Unterseite **2** und eine Öffnung **3** auf. In der Nähe der Öffnung **3** ist die erste, ringförmig, peripher umlaufende Vertiefung **5** ausgebildet und dahinter die in den Dimensionen identische Vertiefung **6**. Dahinter befindet sich ein Wulst **4** auf dem die Öffnung der Kapselkappe **7** im geschlossen Zustand aufliegen kann. Optional kann auf dem Mantel des Kapselunterteils ein Feld **15** ausgebildet sein, auf dem Buchstabencode und/oder Zahlencode aufgebracht werden kann.

In Figur 2 ist eine Ausführungsform der Kaspelkappe **7** dargestellt. Auch die Kappe weist eine konvexe Unterseite **8** und eine Öffnung **9** auf. In der Nähe der Öffnung **9** ist die erste, ringförmig, peripher umlaufende, aber segmentierte Erhebung **11** ausgebildet, der Vorverschluss und dahinter die in den Dimensionen nicht identische, ringförmig, peripher umlaufende, aber nicht-segmentierte Erhebung **10**, der Hauptverschluss. Der Vorverschluss ist als unterbrochener Ring aus drei sich erhebenden länglichen Segmenten **11** und dazwischen liegenden drei, mit dem Mantel der Kappe planaren Segmenten **12** ausgebildet. Die Länge der sich erhebenden Segmente - die Länge verläuft senkrecht zur Kappenachse, der Achse vom geschlossenen Unterteil zur Öffnung - ist länger als die Länge der unterbrechenden Bereiche.

In Figur 3 findet sich eine vergleichbare Kappenausführung, wobei hier nur zwei Segmente in Form von punktähnlichen Erhebungen **13** ausgebildet sind.

In Figur 4 schließlich ist eine Ausführungsform dargestellt, bei der auch der Vorverschluss als durchgehende Erhebung **14** dargestellt ist. Letztere ist in ihren Dimensionen nicht-identisch mit der Erhebung **10.**

Die dargestellten Kapselkappen und der Kapselkörper sind nicht maßstabsgerecht zueinander dargestellt. In Wirklichkeit passen die beiden Bauteile wie komplementäre Bauteil zusammen.

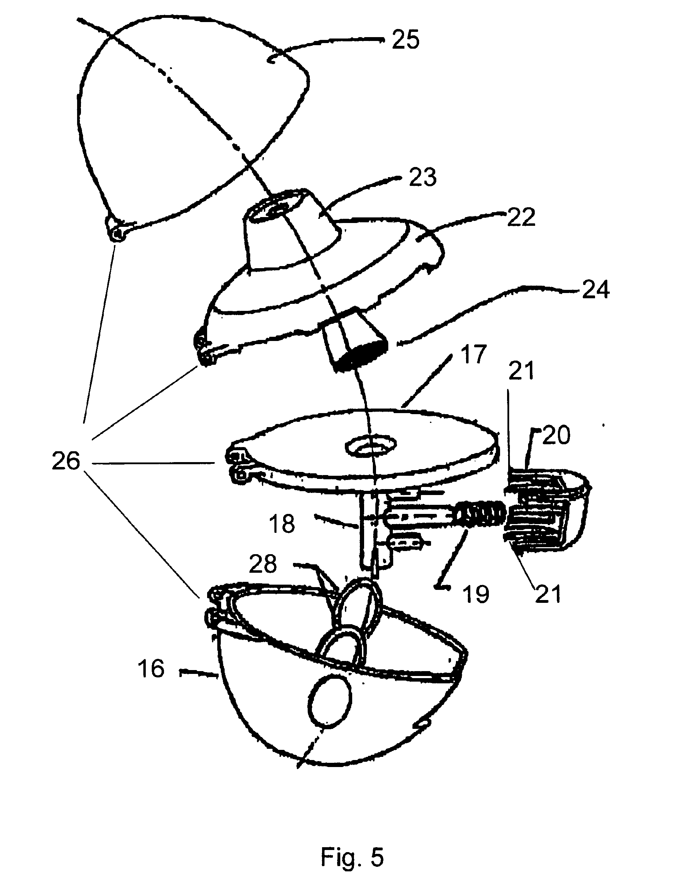

Fig. 5 zeigt den HandiHaler^{®} bestehend aus a) einem nach oben hin offenen, becherförmigen Unterteil **16**, b) einer Platte **17**, welche die Öffnung des Unterteils bedeckt und senkrecht zu der eine Kapselkammer **18** ausgebildet ist, wobei an der Kapselkammer **18** ein gegen eine Feder **19** beweglicher Knopf **20** vorgesehen ist, der zwei geschliffene Nadeln **21** zum Öffnen der Kapsel aufweist, c) einem Oberteil **22** mit einem Mundrohr **23** und einem Lufteinlassrohr mit Sieb **24**, über die das Pulveraerosol aus der Kapselkammer inhaliert werden kann und d) einem Deckel **25**. Dabei sind die Elemente a), b) c) und d) durch ein gemeinsames Scharnierelement **26** miteinander verbunden, so dass sie gegeneinander klappbar bewegt werden können. Das Gerät kann optional die Sichtfenster **28** aufweisen.

## Patentansprüche

1. Kapsel zur Verwendung als Reservoir für eine pharmazeutische Zubereitungen in Pulverinhalatoren bestehend aus zwei einseitig offenen Kapselelementen, einem Kapselkörper und einer Kapselkappe, die teleskopartig über ihre Öffnungen ineinander gesteckt werden können, dass ein stabiler, abgeschlossener Hohlraum von definiertem Volumen gebildet wird, **dadurch gekennzeichnet, dass**
- auf der Innen- oder Außenseite des einen Kapselelements in der Nähe dessen Öffnung zwei hintereinander liegende, peripher den Mantel des Kapselelements umlaufende ringförmige Vertiefungen ausgebildet sind und
- auf dem Außen- oder Innenmantel des anderen Kapselelements in der Nähe dessen Öffnung zwei hintereinander liegende, peripher den Mantel des Kapselkörpers umlaufende ringförmige, ggf. unterbrochene Erhebungen ausgebildet sind,
- wobei die Erhebungen und die Vertiefungen beim teleskopartigen Verschließen der beiden Kapselelemente ineinander einrasten,
- wobei die von der Öffnung aus gesehene erste Erhebung des einen Kapselelements und die erste Vertiefung auf dem anderen Kapselelement durch Einrasten einen Vorverschluss bilden, für dessen Öffnen durch Auseinaderzeihen der beiden Kapselelemente eine erste Kraft notwendig ist, und
- wobei die von der Öffnung aus gesehene zweite Erhebung des einen Kapselelements und die erste Vertiefung auf dem anderen Kapselelement zusammen mit der ersten Erhebung des einen Kapselelements und der zweite Vertiefung auf dem anderen Kapselelement durch jeweiliges Einrasten einen Hauptverschluss bilden, für dessen Öffnen durch Auseinanderziehen der beiden Kapselelemente eine zweite Kraft notwendig ist
- und die erste zum Öffnen aufzubringende Kraft kleiner ist als die zweite Kraft.

2. Kapsel nach Anspruch 1, **dadurch gekennzeichnet,** die erste Kraft bis zu 3 N beträgt.

3. Kapsel nach Anspruch 1 oder 2, **dadurch gekennzeichnet,** der Hauptverschluss durch Auseinanderziehen der Kapselelemente nicht zerstörungsfrei geöffnet werden kann.

4. Kapsel nach einem der Ansprüche 1 bis 3, **dadurch gekennzeichnet, dass** die zweite Erhebung höher und/oder breiter ist und/oder mehr Oberfläche besitzt als die erste Erhebung.

5. Kapsel nach einem der Ansprüche 1 bis 4, **dadurch gekennzeichnet, dass** die Vertiefungen auf der Außenseite des Kapselkörpers und die Erhebungen auf dem Innenmantel der Kapselkappe ausgebildet sind.

6. Kapsel nach einem der Ansprüche 1 bis 5, **gekennzeichnet durch** eine segmentierte erste Erhebung, bevorzugt in Form dreier symmetrisch angeordneter Teilstücke.

7. Kapsel nach einem der Ansprüche 1 bis 6, **dadurch gekennzeichnet, dass** die erste Erhebung der Kapselkappe als eine punktförmige Erhebung ausgebildet ist und der restliche Bestandteil der ringförmigen Form nicht ausgebildet ist.

8. Kapsel nach Anspruch 1 bis 7, **gekennzeichnet durch** eine Tiefe der Vertiefungen von 0,03 bis 0,1 mm, bevorzugt von 0,05 bis 0,05 mm, stärker bevorzugt von 0,065mm.

9. Kapsel nach Anspruch 1 bis 8, **dadurch gekennzeichnet, dass** die erste Erhebung eine Höhe von 0,04 bis 0,08 mm, bevorzugt 0,06 mm aufweist.

10. Kapsel nach Anspruch 1 bis 9, **dadurch gekennzeichnet, dass** die zweite Erhebung eine Höhe von 0,08 bis 0,13 mm, bevorzugt 0,10 -0,11 mm aufweist.

11. Kapsel nach Anspruch 1 bis 10, **dadurch gekennzeichnet, dass** die Wände der Kappe und des Körpers 0,1 mm bis 0,5 mm stark sind.

12. Kapsel nach Anspruch 1 bis 11, **dadurch gekennzeichnet, dass** die Kapselelemente aus Polyethylen, Polycarbonat, Polyester, Polypropylen oder Polyethylenterrephthalat sind.

13. Verwendung der Kapsel nach einem der vorhergehenden Ansprüche 1 bis 12 in Pulverinhalatoren.

## Claims

1. Capsule for use as reservoir for pharmaceutical preparations in powder inhalers consisting of two capsule elements open at one end, a capsule body and a capsule top which can be inserted telescopically into each other via their openings so as to form a stable, sealed cavity of defined volume, **characterized in that**
- two annular channels lying behind one another, running peripherally round the casing of the capsule element, are formed on the inside or outside of the one capsule element in the vicinity of its opening and
- two annular optionally interrupted channels lying behind one another, running peripherally round the casing of the capsule body, are formed on the outer or inner casing of the other capsule element in the vicinity of its opening,
- the raised sections and the channels latching in one another upon the telescopic closure of the two capsule elements,
- wherein the first raised section on the one capsule element, viewed from the opening, and the first channel on the other capsule element form a pre-seal by latching together which requires a first force to open it by pulling apart the two capsule elements, and
- the second raised section of the one capsule element, viewed from the opening, and the first channel on the other capsule element together with the first raised section of the one capsule element and the second channel on the other capsule element form a main closure by respective latching, which requires a second force to open it by pulling apart the two capsule elements,
- and the first force to be applied for opening is less than the second force.

2. Capsule according to claim 1, **characterized in that** the first force is up to 3 N.

3. Capsule according to claim 1 or 2, **characterized in that** the main closure cannot be opened by pulling apart the capsule elements without being destroyed.

4. Capsule according to one of claims 1 to 3, **characterized in that** the second raised section is higher and/or wider and/or has more surface area than the first raised section.

5. Capsule according to one of claims 1 to 4, **characterized in that** the channels are formed on the outside of the capsule body and the raised sections on the inner casing of the capsule top.

6. Capsule according to one of claims 1 to 5, **characterized by** a segmented first raised section, preferably in the form of three symmetrically arranged part-sections.

7. Capsule according to one of claims 1 to 6, **characterized in that** the first raised section of the capsule top is formed as a punctiform raised section and the remainder of the annular shape is not formed.

8. Capsule according to claims 1 to 7, **characterized by** a depth of the channels of 0.03 to 0.1 mm, preferably 0.05 to 0.05 mm, more preferably 0.065mm.

9. Capsule according to claims 1 to 8, **characterized in that** the first raised section is 0.04 to 0.08 mm, preferably 0.06 mm high.

10. Capsule according to claims 1 to 9, **characterized in that** the second raised section is 0.08 to 0.13 mm, preferably 0.10 -0.11 mm high.

11. Capsule according to claims 1 to 10, **characterized in that** the walls of the top and of the body are 0.1 mm to 0.5 mm thick.

12. Capsule according to claims 1 to 11, **characterized in that** the capsule elements are made of polyethylene, polycarbonate, polyester, polypropylene or polyethylene terephthalate.

13. Use of the capsule according to one of the preceding claims 1 to 12 in powder inhalers.

## Revendications

1. Gélule destinée à servir de réservoir de préparations pharmaceutiques dans des inhalateurs à poudre, se composant de deux éléments de gélule ouverts d'un seul côté, d'un corps de gélule et d'un capuchon de gélule qui peuvent être emboîtés de façon télescopique les uns dans les autres par leurs ouvertures de sorte qu'une cavité stable et fermée d'un volume défini est formée, **caractérisée en ce que**
- deux dépressions circulaires entourant de façon périphérique l'enveloppe de l'élément de gélule et se trouvant l'une derrière l'autre sont formées sur la face intérieure ou extérieure de l'un des éléments de gélule à proximité de son ouverture et
- deux bosses circulaires, le cas échéant interrompues, entourant de façon périphérique l'enveloppe du corps de gélule et se trouvant l'une derrière l'autre sont formées sur la face extérieure ou intérieure de l'autre élément de gélule à proximité de son ouverture,
- les bosses et les dépressions s'engageant les uns dans les autres lors de la fermeture télescopique des deux éléments de gélule,
- la première bosse vue à partir de l'ouverture de l'un des éléments de gélule et la première dépression sur l'autre élément de gélule formant une pré-fermeture par engagement, dont l'ouverture par traction et séparation des deux éléments nécessite l'application d'une première force, et
- la deuxième bosse vue à partir de l'ouverture de l'un des éléments de gélule et la première dépression sur l'autre élément de gélule formant respectivement avec la première bosse de l'un des éléments de gélule et la deuxième dépression sur l'autre élément de gélule une fermeture principale par engagement, dont l'ouverture par traction et séparation des deux éléments de gélule nécessite l'application d'une seconde force,
- et **en ce que** la première force appliquée pour l'ouverture étant plus petite que la seconde force.

2. Gélule selon la revendication 1, **caractérisée en ce que** la première force est inférieure ou égale à 3 N.

3. Gélule selon la revendication 1 ou 2, **caractérisée en ce que** la fermeture principale ne peut pas être ouverte de façon non destructive par traction et séparation des deux éléments de gélule.

4. Gélule selon l'une des revendications 1 à 3, **caractérisée en ce que** la seconde bosse est plus élevée et/ou plus large et/ou a une plus grande de surface que la première bosse.

5. Gélule selon l'une des revendications 1 à 4, **caractérisée en ce que** les dépressions sont formées sur la face extérieure du corps de la gélule et les bosses sur l'enveloppe intérieure du capuchon de gélule.

6. Gélule selon l'une des revendications 1 à 5, **caractérisée par** une première bosse segmentée, de préférence sous la forme de trois sections disposées symétriquement.

7. Gélule selon l'une des revendications 1 à 6, **caractérisée en ce que** la première bosse du capuchon de gélule est conçue comme une bosse en forme de point et **en ce que** le reste de la forme circulaire n'est pas formé.

8. Gélule selon la revendication 1 à 7, **caractérisée en ce que** les dépressions ont une profondeur de 0,03 à 0,1 mm, de préférence de 0,05 à 0,08 mm, de manière encore plus fortement préférée de 0,065 mm.

9. Gélule selon la revendication 1 à 8, **caractérisée en ce que** la première bosse présente une hauteur de 0,04 à 0,08 mm, de préférence de 0,06 mm.

10. Gélule selon la revendication 1 à 9, **caractérisée en ce que** la deuxième bosse présente une hauteur de 0,08 à 0,13 mm, de préférence de 0,10 à 0,11 mm.

11. Gélule selon la revendication 1 à 10, **caractérisée en ce que** les parois du capuchon et du corps présentent une épaisseur de 0,1 mm à 0,5 mm.

12. Gélule selon la revendication 1 à 11, **caractérisée en ce que** les éléments de gélule sont en polyéthylène, polycarbonate, polyester, polypropylène ou polyéthylènetéréphthalate.

13. Utilisation de la gélule selon l'une quelconque des revendications précédentes 1 à 12 dans des inhalateurs à poudre.
